# FASCICULE DE BREVET EUROPEEN

(11) **EP 1 284 984 B1**
(45) Date de publication et mention de la délivrance du brevet: **02.11.2006**
(21) Numéro de dépôt: 01931788.2
(22) Date de dépôt: 04.05.2001
(51) Int. Cl.: C07H 19/06, A61K 31/70, A61P 31/04

(54) **DERIVES DE L'URIDINE COMME ANTIBIOTIQUES**
URIDINDERIVATE ALS ANTIBIOTIKA
URIDINE DERIVATIVES AS ANTIBIOTICS

(30) Priorité: 09.05.2000 FR 0005858
(43) Date de publication de la demande: 26.02.2003
(73) Titulaire: Aventis Pharma S.A., 92160 Antony (FR)
(72) Inventeur: ASZODI, Jozsef, F-77340 Pontault Combault (FR); DIDIERLAURENT, Stanislas, F-77400 Lagny-sur-Marne (FR); DINI, Christophe, F-91220 Le Plessis Pate (FR); DROCHON, Nathalie, F-93110 Rosny sous Bois (FR); GUILLOT, Jean-Claude, F-93130 Noisy le Sec (FR); ZHANG, Jidong, F-75013 Paris (FR)
(74) Mandataire: Vieillefosse, Jean-Claude
(86) Numéro de dépôt international: PCT/FR2001/001356
(87) Numéro de publication internationale: WO 2001/085750

(56) Documents cités:
- WO-A-97/41248
- PATENT ABSTRACTS OF JAPAN vol. 015, no. 092, 6 mars 1991 (1991-03-06) & JP 02 306992 A (RIKAGAKU KENKYUSHO), 20 décembre 1990 (1990-12-20)
- PATENT ABSTRACTS OF JAPAN vol. 017, no. 406, 29 juillet 1993 (1993-07-29) & JP 05 078385 A (FUJISAWA PHARMACEUTICAL CO., LTD.), 30 mars 1993 (1993-03-30)
- M. UBUTAKU, K. ISONO: "The Structure of Liposidomycin B, an Inhibitor of Bacterial Peptidoglycan Synthesis" J. AM. CHEM. SOC., vol. 110, no. 13, 1988, pages 4416-4417, XP002934331
- K. IMURA ET AL.: "Liposidomycin C Inhibits Phospho-N-acetylmuramyl-pentapeptide Transferase in Peptidoglycan Synthesis of Escherichia coli Y-10" AGRIC. BIOL. CHEM., vol. 53, no. 7, 1989, pages 1811-1815, XP002934329
- S. KNAPP ET AL.: "Synthesis of the Liposidomycin Diazepanone" TETRAHEDRON LETTERS, vol. 33, no. 38, 1992, pages 5485-5486, XP002934328
- M. UBUKATA: "Chemical Studies on New Antibiotics" JOURNAL OF JAPAN SOCIETY FOR BIOSCIENCE, BIOTECHNOLOGY AND AGROCHEMISTRY (JSBA), vol. 62, no. 11, 1988, pages 1629-1636, XP002934330
- M. UBUTAKA ET AL.: "Structure Elucidation of Liposidomycins, a Class of Complex Lipid Nucleoside Antibiotics" J. ORG. CHEM., vol. 57, no. 24, 1992, pages 6392-6403, XP002934327

## Description

La présente invention concerne de nouveaux dérivés de l'uridine, leur procédé de préparation et leur application comme médicaments.

L'invention a pour objet sous toutes les formes isomères possibles ainsi que leurs mélanges, les composés de formule (I) : 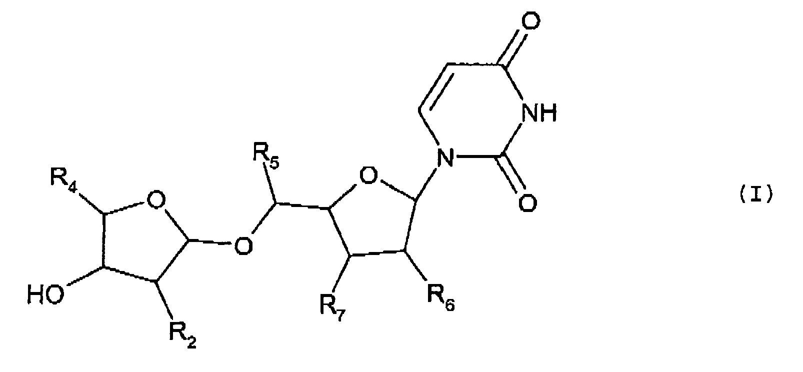
dans lesquels R₂ représente un atome d'hydrogène ou d'halogène, un radical alkyle, alkényle ou alkynyle renfermant jusqu'à 12 atomes de carbone, un radical OH, O-alkyle, OCO-alkyle, renfermant jusqu'à 18 atomes de carbone, un radical O-aryle ou OCO-aryle renfermant jusqu'à 15 atomes de carbone,
R₄ représente un radical CH₂NH₂, CH₂NHalkyle renfermant jusqu'à 18 atomes de carbone, CH₂N(alkyle1)(alkyle2) dans lequel alkyle1 et alkyle2 identiques ou différents l'un de l'autre, représentent un radical alkyle linéaire ou ramifié, renfermant jusqu'à 18 atomes de carbone, ou R₄ représente un radical CH₂-guanidine ou CH₂-amidine, un radical CH₂- hétérocycle, CH₂-NH-hétérocycle ou CH₂-NH-aryle ou CH₂-NH-hétéroaryle éventuellement substitués,
R₅ représente un radical 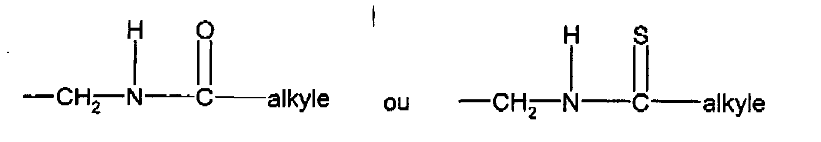 renfermant jusqu'à 24 atomes de carbone, un radical 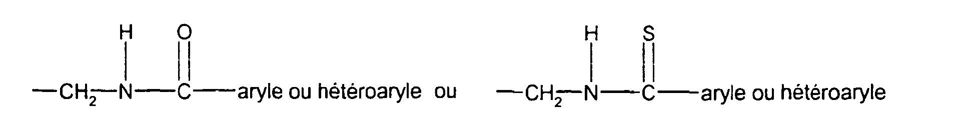 renfermant jusqu'à 24 atomes de carbone, un radical CH₂N₃, un radical : 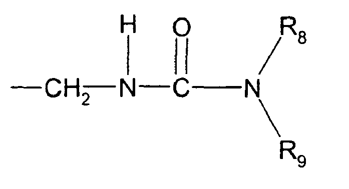 un radical : 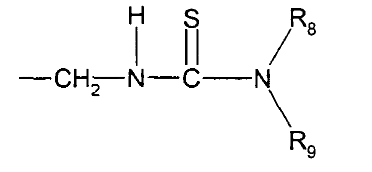 un radical : 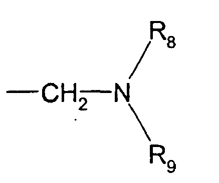
avec R₈ représentant un atome d'hydrogène ou un radical alkyle renfermant jusqu'à 4 atomes de carbone,
et R₉ représentant un radical alkyle, linéaire ou ramifié renfermant au plus 18 atomes de carbone, un radical carbocyclique ou hétérocyclique renfermant 5 ou 6 chaînons, un radical aryle ou hétéroaryle éventuellement substitués, R₈ et R₉ pouvant également constituer avec l'atome d'azote qui les porte un hétérocycle éventuellement substitué, ou R₅ représente un radical CH₂alkyle, CH₂-aryle ou CH₂-hétéroaryle éventuellement substitués, CH₂Oalkyle ou CO₂alkyle renfermant jusqu'à 18 atomes de carbone, ou CH₂S-alkyle renfermant jusqu'à 16 atomes de carbone, ou CH₂S-aryle ou CH₂S-hétéroaryle, éventuellement substitués,
R₆ représente un atome d'hydrogène ou d'halogène, un radical OH, Oalkyle ou OCOalkyle renfermant jusqu'à 18 atomes de carbone, un radical S-alkyle, S-aryle ou S-hétéroaryle renfermant jusqu'à 12 atomes de carbone, tous les radicaux alkyle, aryle, hétéroaryle, les radicaux carbocycliques et hétérocycliques définis ci-dessus étant éventuellement substitués par un ou plusieurs radicaux choisis parmi les atomes d'halogène ; le radical hydroxyle ; les radicaux alkyle ou alcoxy linéaires ou ramifiés, renfermant 1 à 4 atomes de carbone ; le radical phényle et phénylalkyle, le radical phényle étant lui-même éventuellement substitué par un atome d'halogène ou un radical phényle et le radical alkyle linéaire ou ramifié renfermant de 1 à 4 atomes de carbone ; un radical carbocyclique renfermant 4 ou 6 chaînons ;
R₇ représente un atome d'hydrogène ou un radical OH libre, éthérifié ou estérifié,
ainsi que les sels d'addition avec les acides des composés de formule (I) .

Parmi les sels d'addition avec les acides, on peut citer ceux formés avec les acides minéraux, tels que les acides chlorhydrique, bromhydrique, sulfurique ou phosphorique ou avec les acides organiques comme l'acide formique, acétique, trifluoroacétique, propionique, benzoïque, maléique, fumarique, succinique, tartrique, citrique, oxalique, glyoxilique, aspartique, alcanesulfoniques, tels que les acides méthane ou éthane sulfoniques, arylsulfoniques tels que les acides benzène ou paratoluènesulfoniques.

Dans la définition des substituants :
- l'halogène est de préférence le fluor, le brome ou le chlore,

Les radicaux alkyle linéaires ou ramifiés, peuvent contenir jusqu'à 24 atomes de carbone, les radicaux alkényle et alkynyle linéaires ou ramifiés, peuvent renfermer jusqu'à 12 atomes de carbone. Ces radicaux alkyle, alkényle et alkynyle sont choisis parmi les radicaux bien connus de l'homme de métier.
- le radical alkyle, alkényle ou alkynyle peut représenter par exemple un radical méthyle, éthyle, n-propyle, isopropyle, n-butyle isobutyle, terbutyle, décyle ou dodécyle, vinyle, allyle, éthynyle, propynyle, cyclobutyle, cyclopentyle ou cyclohexyle,
- le radical aryle est de préférence le radical phényle ou naphtyle,
- le radical hétérocyclique peut représenter de préférence le radical pyrrolyle, pyrrolidinyle, pyridyle, pyrazinyle, pyrimidyle, pipéridinyle, pipérazinyle, quinuclidinyle, oxazolyle, isoxazolyle, morpholinyle, indolyle, imidazolyle, benzimidazolyle, triazolyle, thiazolyle, azétidinyle ou aziridinyle.

Le radical carbocyclique peut représenter notamment un radical cyclopentyle ou cyclohexyle.

L'invention a plus particulièrement pour objet le composé défini précédemment de formule (IA) : 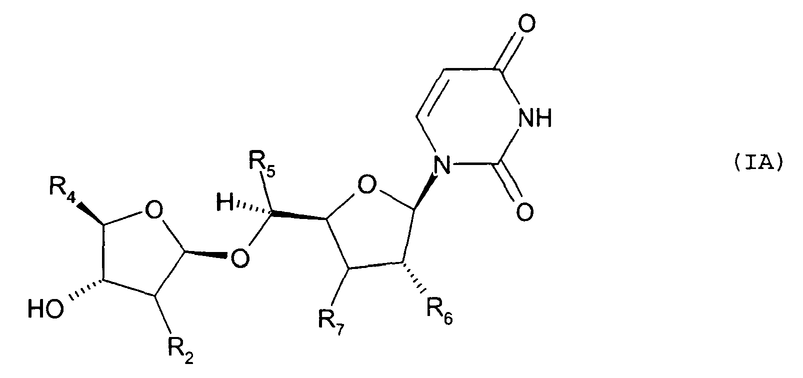 dans lesquels les divers substituants conservent la même signification que précédemment.

Parmi les composés préférés de l'invention on peut citer :
- les composés de formule (I) dans lesquels R₇ représente un atome d'hydrogène,
- les composés de formule (I) dans lesquels R₆ représente un radical OH ou un atome de fluor,
- les composés de formule (I) dans lesquels R₂ représente un radical OH,
- les composés de formule (I) dans lesquels R₂ représente un atome de fluor,
- les composés de formule (I) dans lesquels R₄ représente un radical CH₂NH₂,
- les composés de formule (I) dans lesquels R₄ représente un radical CH₂NHCH₃ ou CH₂NHC₂H₅,
- les composés de formule (I) dans lesquels R₅ représente un radical 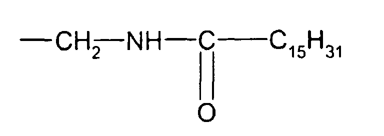
- les composés de formule (I) dans lesquels R₅ représente un radical 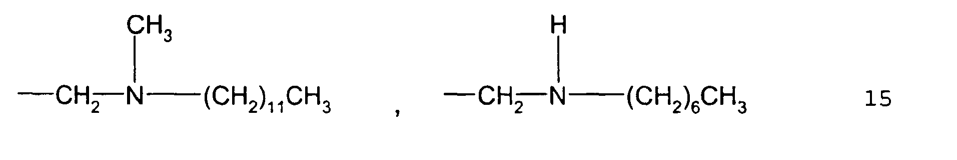 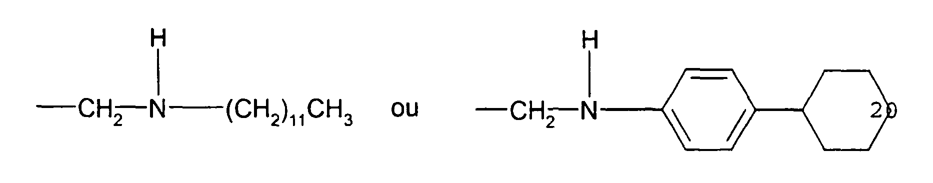
ou CH₂-NH-alkyle avec alkyle linéaire ou ramifié renfermant 8 à 14 atomes de carbone.

Parmi les composés de l'invention, on peut citer les composés dont la préparation est donnée ci-après dans la partie expérimentale et tout spécialement le produit de l'exemple 1 et ses sels. On peut citer par exemple le trifluoroacétate de l'exemple 1.

Les composés de l'invention présentent d'intéressantes propriétés antibiotiques. Ils possèdent une très bonne activité antibiotique notamment sur les bactéries gram^{⊕} telles que les staphylocoques, les streptocoques, les pneumocoques et les entérocoques.

Les composés de l'invention peuvent donc être utilisés comme médicaments dans le traitement des infections à germes sensibles et notamment, dans celui des staphylococcies malignes de la face ou cutanées, pyodermites, plaies septiques ou suppurantes, furoncles, anthrax, phlegmons, érysipèles et acné, staphylococcies telles que les angines aiguës primitives ou post-grippales, bronchopneumonies, suppuration pulmonaires, les streptococcies telles que les angines aiguës, les otites, les sinusites, la scarlatine, les pneumococcies telles que les pneumonies, les bronchites, la brucellose, la diphtérie, la gonococcie.

Les produits de la présente invention peuvent également être utilisés contre les infections dues à des germes comme Haemophilus influenzae, Rickettsies, Mycoplasma pneumoniae, Clamydia, Legionella, Ureaplasma, Toxoplasma ou à des germes du genre Mycobactérium.

La présente invention a donc également pour objet, à titre de médicaments et, notamment de médicaments antibiotiques, les produits de formule (I) tels que définis ci-dessus, ainsi que leurs sels d'addition avec les acides minéraux ou organiques pharmaceutiquement acceptables.

L'invention a plus particulièrement pour objet, à titre de médicament et, notamment de médicament antibiotique le produit de l'exemple 1 et ses sels pharmaceutiquement acceptables.

L'invention a également pour objet les compositions pharmaceutiques renfermant comme principe actif au moins un des médicaments définis ci-dessus.

Ces compositions peuvent être administrées par voie buccale, rectale, parentérale ou par voie locale en application topique sur la peau et les muqueuses, mais la voie d'administration préférée est la voie buccale ou parentérale.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 300 mg par jour par voie orale, chez l'adulte pour le produit de l'exemple 1 en une ou plusieurs prises.

L'invention a également pour objet un procédé de préparation des composés de formule (I) caractérisé en ce que l'on soumet un composé de formule (II) : 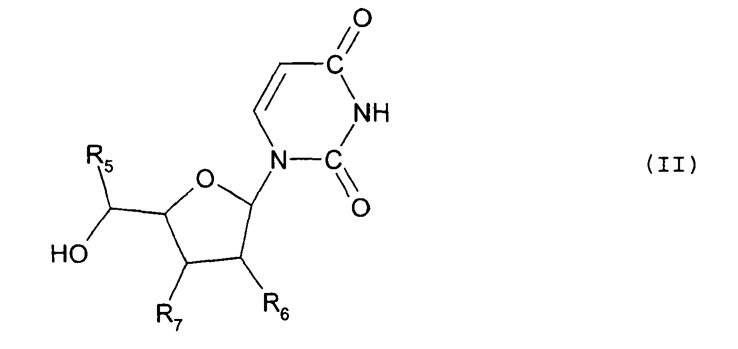 dans laquelle les divers substituants conservent leur signification précédente, à l'action d'un composé de formule (III) : 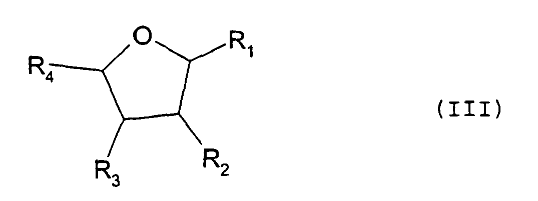 dans laquelle R₁ représente un atome d'halogène ou un radical OC(NH)C(Hal)₃ avec Hal représentant un atome d'halogène ou un radical 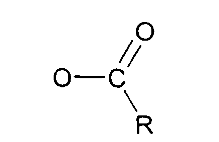 avec R représentant un radical alkyle renfermant jusqu'à 18 atomes de carbone les autres substituants R₂, R₃ et R₄ conservent leur signification précédente pour obtenir le composé de formule (I) correspondant que l'on soumet si désiré à une ou plusieurs des opérations suivantes : addition, substitution, condensation et salification pour obtenir le composé de formule (I) recherché.

Les composés de formule (II) utilisés comme produits de départ sont des produits nouveaux qui peuvent être préparés comme indiqué ci-après dans la partie expérimentale.

Les exemples suivants illustrent l'invention sans toutefois la limiter.

### EXEMPLE 1 : 1-[5-O-(5-amino-5-déoxy-.béta.-D-ribofuranosyl)-3,6-didéoxy-6-[(1-oxohexadécyl)amino]-.béta.-D-ribohexofuranosyl]-2,4-(1H,3H)-pyrimidinedione

### STADE A : 1-[2-O-acétyl-3,6-didéoxy-5-O-[(4-méthoxyphényl) méthyl]-6-[(1-oxohexadécyl)amino]-.béta.-D-ribohexofuranosyl]-2,4-(1H,3H)-pyrimidinedione

On agite sous atmosphère d'hydrogène pendant 16 heures, un mélange renfermant 450 mg de produit stade E, préparé comme indiqué ci-dessous (préparation 1), 25 ml d'acétate d'éthyle, 590 mg d'anhydride palmitique et 120 mg de palladium sur charbon. On filtre, évapore et sèche. On obtient 940 mg de produit que l'on utilise tel quel dans le stade suivant.

### STADE B : 1-[2-O-acétyl-3,6-didéoxy-6-[(1-oxohexadécyl) amino]-.béta.-D-ribo-hexofuranosyl]-2,4-(1H,3H)-pyrimidinedione

On ajoute 240 mg de produit du stade A, dissous dans 2 ml de chlorure de méthylène dans 7,5 ml d'un mélange de chlorure de méthylène/acide trifluoroacétique (9/1), sous atmosphère d'azote. On agite la solution obtenue pendant 1 heure. On évapore et sèche. On obtient un produit que l'on reprend dans l'éther puis dans le pentane. On évapore à nouveau. On chromatographie le produit obtenu et obtient 47 mg de produit recherché.

### STADE C : 1-[2-O-acétyl-5-O-(5-azido-2,3-di-O-acétyl-5-déoxy.béta.-D-ribofuranosyl)-3,6-didéoxy-6-[(1-oxohexadécyl)amino] .béta.-D-ribo-hexofuranosyl]-2,4-(1H,3H)-pyrimidinedione

On ajoute à 0°C 330 µl de SnCl₄ (solution 1M dans CH₂Cl₂) dans un mélange de 120 mg de produit du stade précédent, 132 mg de 1,2,3-triacétate de 5-azido-5-déoxy-.béta.-D-ribofuranose et 1 ml de chlorure de méthylène, on agite une nuit à température ambiante. On ajoute 3 ml de chlorure de méthylène et 2 ml de carbonate acide de sodium. On agite 15 minutes à la température ambiante. On filtre, on sépare la phase organique et sèche. On évapore, chromatographie sur silice, et récupère 64 mg de produit recherché que l'on utilise tel quel dans le stade suivant.

### STADE D : 1-[5-O-(5-azido-5-déoxy-.béta.-D-ribofuranosyl)-3,6-didéoxy-6- [(1-oxohexadécyl)amino] -.béta.-D-ribohexofuranosyl]-2,4-(1H,3H)-pyrimidinedione

On maintient sous agitation pendant 15 heures, un mélange de 64 mg de produit du stade précédent et 2 ml d'une solution de méthylate de sodium 0,1M dans le méthanol. On ajoute 2 ml de méthanol et de la résine IR 120H⁺. On filtre, évapore et obtient 52 mg de produit recherché.

### STADE E : 1-[5-O-(5-amino-5-déoxy-.béta.-D-ribofuranosyl)-3,6-didéoxy-6- [(1-oxohexadécyl)amino]-.béta.-D-ribohexofuranosyl]-2,4-(1H,3H)-pyrimidinedione

On agite sous atmosphère d'hydrogène, pendant 3 heure 30 minutes, un mélange de 20 mg du produit du stade précédent, 1 ml de méthanol, 26,5 mg de palladium sur charbon à 15 %. On filtre, rince et concentre. On obtient 16 mg de produit recherché.

### EXEMPLE 2 : (4-méthylphényl)sulfonate de 1-[6-azido-5-O-[2,5-didéoxy-5-(éthylamino)-2-fluoro-.béta.-D-arabinofuranosyl]-2-fluoro-2,3,6-tridéoxy-.béta.-D-ribo-hexofuranosyl]-2,4-(1H,3H)-pyrimidinedione

### STADE A : 3,5-dibenzoate de 2-déoxy-2-fluoro-D-arabinose

On ajoute 100 ml d'une solution de carbonate acide de sodium à 5 % dans l'eau, dans un mélange de 9,05 g de 3,5-dibenzoate de bromure de 2-déoxy-2-fluoro-alpha-D-arabinose et 90 ml d'acétone. On ajoute 50 ml d'eau. On agite pendant 3 h 30. On dilue, extrait avec du chlorure de méthylène, sèche, filtre et évapore à sec.

### STADE B : 3,5-dibenzoate et 1-(2,2,2-trichloroéthanimidate) de 2-déoxy-2-fluoro-D-arabinose

On maintient sous agitation pendant 2 heures, un mélange de 7,4 g de produit du stade A, 670 mg de carbonate de cesium et 4,12 ml de trichloroacétonitrile. On filtre en rinçant avec du chlorure de méthylène et évapore le solvant. On obtient 9,45 g de produit recherché.

### STADE C : 1-[6-azido-5-O-(2-déoxy-3,5-di-O-benzoyl-2-fluoro-.béta.-D-arabinofuranosyl)-2-fluoro-2,3,6-tridéoxy-.béta.-D-ribo-hexofuranosyl]-2,4-(1H,3H)-pyrimidinedione

On refroidit à -30°C un mélange de 715 mg de produit du stade précédent et 270 mg de 1-(6-azido-2-fluoro-2,3,6-tridéoxy-béta-D-ribo-hexofuranosyl)-2,4-(1H,3H)-pyrimidinedione (préparation 2) et 10 ml de chlorure de méthylène. On ajoute 205 *µ*l de TMSOTf (Trifluorométhanesulfonate de triméthylsilyle). On agite pendant 6 heures. On ajoute à 0°C une solution de carbonate acide de sodium, extrait et récupère la phase organique sur le chlorure de méthylène. On sèche, filtre et évapore le solvant. On obtient un produit que l'on chromatographie sur silice en éluant avec le mélange chlorure de méthylène/acétate d'éthyle (8/2). On obtient 152 mg de produit recherché.

### STADE D : 1-[6-azido-5-O-(2-déoxy-2-fluoro-.béta.-D-arabinofuranosyl)-2-fluoro-2,3,6-tridéoxy-.béta.-D-ribohexofuranosyl]-2,4-(1H,3H)-pyrimidinedione

On reprend 152 mg du produit du stade précédent dans 2 ml d'une solution de MeONa/MeOH 0,1 M sous agitation magnétique à température ambiante. On ajoute IR 120 H⁺ jusqu'à pH neutre. On filtre en rinçant avec du méthanol et évapore le méthanol. On purifie par filtration sur cartouche de silice en éluant avec du chlorure de méthylène, puis avec un mélange chlorure de méthylène/méthanol (9/1). On obtient 98 mg de produit recherché.

### STADE E : 1-[6-azido-5-O-[2-déoxy-2-fluoro-5-O-[(4-méthylphényl)sulfonyl]-.béta.-D-arabinofuranosyl]-2-fluoro-2,3,6-tridéoxy-.béta.-D-ribo-hexofuranosyl]-2,4-(1H,3H)-pyrimidinedione

On solubilise 403 mg du produit du stade précédent dans 2 ml de pyridine sous atmosphère d'argon et agitation magnétique. On ajoute 225 mg de chlorure de tosyle. On lave avec une solution d'hydrogénosulfate de sodium à 10 %, puis au carbonate acide de sodium puis une solution aqueuse de NaHCO₃ saturée, puis à l'eau. On filtre, sèche sur sulfate de sodium. On chromatographie sur silice en éluant avec le mélange chlorure de méthylène/méthanol (97/3). On obtient 360 mg de produit recherché.

### STADE F : (4-méthylphényl)sulfonate de 1-[6-azido-5-O-[2,5-didéoxy-5-(éthylamino)-2-fluoro-.béta.-D-arabinofuranosyl]-2-fluoro-2,3,6-tridéoxy-.béta.-D-ribo-hexofuranosyl]-2,4-(1H,3H)-pyrimidinedione

On maintient à 60°C pendant 18 heures, un mélange de 80 mg du produit précédent, 2 ml d'une solution aqueuse d'éthylamine à 33 %. On chromatographie le produit obtenu sur silice en éluant avec le mélange chlorure de méthylène/méthanol (8/2). On obtient 37 mg de produit recherché.

### PREPARATION 1 : 1-[6-azido-3,6-didéoxy-5-O-[(4-méthoxyphényl) éthyl]-.béta.-D-ribo-hexofuranosyl]-2,4-(1H,3H)-pyrimidine-ione

### STADE A : 6-azido-3,6-didéoxy-1,2-O-(1-méthyléthylidène)-.alpha.-D-ribo-hexofuranose

On chauffe à 60°C pendant 15 minutes, 200 mg de 5,6-anhydro-3-déoxy-1,2-O-(1-méthyléthylidéne)-.alpha.-D-ribohexofuranose dissous dans 2 ml de DMF. On ajoute 350 µl de méthanol et 300 µl de TMSN₃. On agite à 60°C pendant 48 heures, refroidit, dilue au chlorure de méthylène, lave et sèche. On obtient un produit que l'on reprend dans le méthanol. On ajoute de la résine, IRA 120H⁺, on agite 10 minutes à température ambiante, puis on filtre, rince et concentre à sec. On obtient 230 mg du produit recherché. STADE B : 6-azido-3,6-didéoxy-5-O-[(4-méthoxyphényl)méthyl]-1,2-O-(1-méthyléthylidène)-.alpha.-D-ribo-hexofuranose

On ajoute à 0°C, 6,27 g d'hydrure de sodium dans une solution renfermant 20 g de produit préparé au stade précédent et dissous dans le DMF. On ajoute 14,15 ml de PMBC1 (chlorure de paraméthoxybnezyle)et on agite 1 heure à température ambiante. On refroidit à 0°C et ajoute 3 ml de méthanol. On agite 30 minutes à température ambiante. On filtre, rajoute du chloroforme et concentre à sec. On obtient 31,7 g de produit recherché.

### STADE C : 6-azido-3,6-didéoxy-5-O-[(4-méthoxyphényl)méthyl]-.alpha.-D-ribo-hexofuranose

On agite à 40°C pendant 24 heures, une solution renfermant 34,8 g de produit du stade B et 500 ml d'une solution aqueuse d'acide acétique à 50 %. On agite pendant 36 heures à 40°C. On dilue avec un litre d'eau, extrait à l'acétate d'éthyle, sèche, filtre et évapore. On chromatographie sur silice en éluant avec le mélange chlorure de méthylène/acétate d'éthyle (99,5/0,5) et on récupère 12,5 g de produit attendu.

### STADE D : 1,2-diacétate de 6-azido-3,6-didéoxy-5-O-[(4-méthoxyphényl)méthyl]-D-ribo-hexofuranose

On agite sous atmosphère d'argon une solution de 10 g de produit du stade précédent dans 200 ml de pyridine. On ajoute 130 ml d'anhydride acétique. On agite 2 heures, refroidit à 0°C pendant 15 minutes, ajoute 200 ml de méthanol. On évapore le solvant, obtient un produit que l'on reprend dans le chlorure de méthylène, lave avec de l'acide chlorhydrique 0,1 M et avec une solution aqueuse de chlorure de sodium. On filtre, sèche et évapore le chlorure de méthylène, on obtient 12,43 g de produit recherché.

### STADE E : 1-[2-O-acétyl-6-azido-3,6-didéoxy-5-O-[(4-méthoxyphényl)méthyl]-.béta.-D-ribo-hexofuranosyl]- 2,4-(1H,3H)-pyrimidinedione

On maintient sous agitation et atmosphère d'argon pendant une heure, un mélange de 3,58 g d'uracile, 120 ml d'acétonitrile, 8 ml de BSA N-O, bis-triméthylsilylacétamide et 11,43 g de produit du stade précédent en solution dans 275 ml d'acétonitrile. On ajoute 9,58 ml de bromure de triméthylsilyle. On maintient sous agitation pendant 18 heures, ajoute 20 ml de triéthylamine. On évapore l'acétonitrile. On reprend dans le chlorure de méthylène, lave avec une solution aqueuse 1M de chlorure de méthylène, puis avec une solution aqueuse de chlorure de sodium. On sèche et concentre à sec. On filtre sur silice en éluant avec le mélange chlorure de méthylène/méthanol (98/2). On obtient 9,98 g de produit recherché.

### PREPARATION 2 : 1-(6-azido-2-fluoro-2,3,6-tridéoxy-.béta.-D-ribo-hexofuranosyl)-2,4-(1H,3H)-pyrimidinedione

### STADE A : 1-[6-azido-3,6-didéoxy-5-O-[(4-méthoxyphényl) éthyl]-.béta.-D-ribo-hexofuranosyl]-2,4-(1H,3H)-pyrimidine-ione

On dissout 10 g de produit de la préparation 1 dans 300 ml d'une solution 0,1 M de méthylate de sodium dans le méthanol. On agite pendant 15 minutes. On amèe à pH 6, filtre, rince au méthanol et évapore le méthanol. On obtient 8,7 g de produit recherché.

### STADE B : 1-[6-azido-3,6-didéoxy-5-O-[(4-méthoxyphényl) méthyl]-2-O-(méthylsulfonyl)-.béta.-D-ribo-hexofuranosyl]-2,4-(1H,3H)-pyrimidinedione

On dissout 8,7 g de produit préparé au stade précédent dans 200 ml d'acétone. On ajoute à 0°C 5,9 ml de triéthylamine et 2,5 ml de chlorure de mésyle. On agite pendant 1 heure à 0°C et ajoute 5,9 ml de triéthylamine et 2,5 ml de chlorure de mésyle et on laisse sous agitation 2 heures supplémentaires. On évapore l'acétone, reprend à l'acétate d'éthyle, lave avec une solution d'acide chlorhydrique 1M puis avec une solution aqueuse de chlorure de sodium. On sèche et évapore l'acétate d'éthyle. On obtient 10,63 g de produit recherché.

### STADE C : [2R-(2.alpha., 3a.béta., 9a.béta.)]-2-[2-azido-1(S)-[(4-méthoxyphényl)méthoxy]éthyl]-6H-furo[2',3':4,5]oxazolo [3, 2-a] pyrimidin-6-one

On dissout 10,63 g de produit du stade précédent dans 400 ml d'acétinitrile. On ajoute 5 ml de DBU. Après 30 minutes, on évapore l'acétonitrile, lave avec une solution aqueuse d'acide chlorhydrique 0,1M, puis avec du carbonate acide de potassium, puis à l'eau. On filtre, évapore le solvant. On obtient 9,53 g de produit recherché.

### STADE D : 1-[6-azido-3,6-didéoxy-5-O-[(4-méthoxyphényl) méthyl]-.béta.-D-lyxo-hexofuranosyl]-2,4-(1H,3H)-pyrimidinedione

On ajoute 150 ml d'une solution de soude 1M dans une solution de 9,53 g de produit du stade précédent dans 400 ml d'acétonitrile. On ajoute 150 ml de soude 1M. On agite pendant une nuit. On ajoute une solution d'acide chlorhydrique 1M jusqu'à l'obtention d'un pH égal à 7. On évapore l'acétonitrile. On reprend dans l'acétate d'éthyle, lave, sèche, filtre et évapore dans l'acétate d'éthyle. On obtient 9,03 g de produit recherché.

### STADE E : 1-[6-azido-2-fluoro-5-O-[(4-méthoxyphényl)méthyl]-2,3,6-tridéoxy-.béta.-D-ribo-hexofuranosyl]-2,4-(1H,3H)-pyrimidinedione

On dissout 1 g de produit du stade précédent dans 20 ml de chlorure de méthylène. On ajoute 10 ml de pyridine. On refroidit à 0°C et ajoute 6 ml de DAST. On agite pendant 4 heures. On verse le mélange réactionnel dans un mélange de glace et de carbonate acide de sodium. On agite. On filtre et rince au chlorure de méthylène. On lave, extrait, sèche, filtre et évapore le solvant. On obtient un produit que l'on reprend au toluène et évapore pour chasser la pyridine. On chromatographie sur silice en éluant avec le mélange chlorure de méthylène/acétate d'éthyle (8/2) et obtient 1 g de produit recherché.

### STADE F : 1-(6-azido-2-fluoro-2,3,6-tridéoxy-.béta.-D-ribohexofuranosyl)-2,4-(1H, 3H)-pyrimidinedione

On solubilise 2,11 g de produit du stade précédent dans un minimum de chlorure de méthylène et ajoute 70 ml d'une solution de chlorure de méthylène et de TFA (9/1) refroidie à 0°C. On agite pendant une heure et concentre à sec. On sèche et chromatographie sur silice en éluant avec le mélange chlorure de méthylène/acétate d'éthyle (6/4). On obtient 1,09 g de produit.

En opérant comme précédemment pour les exemples 1 et 2, on a préparé les 18 produits représentés dans le tableau suivant qui constituent les exemples 3 à 20 de la présente invention :

| R₂ | R₄ | R₅ | R₆ | R₇ | EX |
|---|---|---|---|---|---|
| OH | CH₂NH₂ | 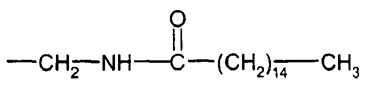 | OH | H | 1 |
| F | -CH₂NHCH₂CH | -CH₂N₃ | F | H | 2 |
| OH | CH₂NH₂ | CH₂NH(CH₂)₆CH₃ | OH | H | 3 |
| OH | CH₂NH₂ | 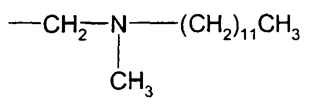 | OH | H | 4 |
| OH | CH₂NH₂ | 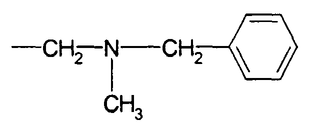 | OH | H | 5 |
| OH | CH₂NH₂ | 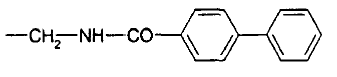 | OH | H | 6 |
| OH | CH₂NH₂ | 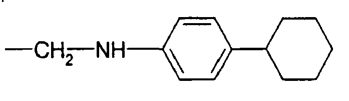 | OH | H | 7 |
| OH | CH₂NH₂ | 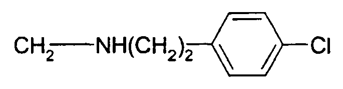 | OH | H | 8 |
| OH | CH₂NH₂ | 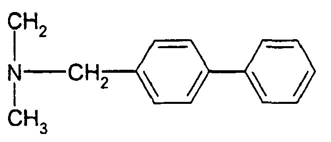 | 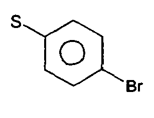 | H | 9 |
| OH | CH₂NH₂ | 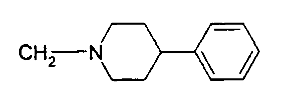 | Cl | H | 10 |
| OH | CH₂NH₂ | 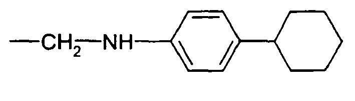 | 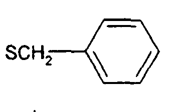 | H | 11 |
| OH | CH₂NH₂ | 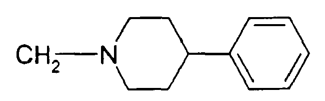 | 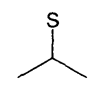 | H | 12 |
| H | CH₂NH₂ | CH₂O(CH₂)₂CH₃ | F | H | 13 |
| OH | CH₂NH₂ | 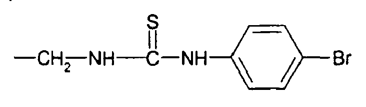 | OH | H | 14 |
| OH | CH₂NH₂ | 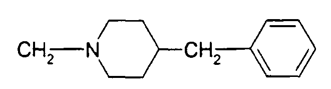 | Cl | H | 15 |
| OH | CH₂NH₂ | 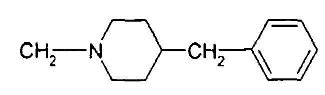 | 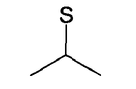 | H | 16 |
| OH | CH₂NH₂ | 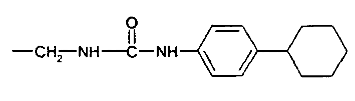 | OH | H | 17 |
| OH | CH₂NH₂ | -CH₂-NH(CH₂)₇-CH₃ | OH | H | 18 |
| OH | CH₂NH₂ | -CH₂-NH(CH₂)₉-CH₃ | OH | H | 19 |
| OH | CH₂NH₂ | -CH₂-NH(CH₂)₁₃-CH₃ | OH | H | 20 |

### EXEMPLE 21 DE COMPOSITIONS PHARMACEUTIQUES

On a préparé des composés de formule (I) répondant à la composition suivante :

| | |
|---|---|
| Produit de l'exemple 1 | 150 mg |
| Excipient q.s.p. | 1 g |

Détail de l'excipient amidon, talc, stéarate de magnésium.

### Etude pharmacologique des produits de l'invention

### Méthode des dilutions en milieu liquide.

On prépare une série de tubes dans lesquels on répartit une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de vingt-quatre heures à l'étuve à 37°C, l'inhibition de la croissante est appréciée par transillumination, ce qui permet de déterminer les concentrations minimales inhibitrices (C.M.I.) exprimés en microgrammes/cm3.

Les produits de l'invention en particulier le produit de l'exemple 1, présentent une bonne activité sur les souches S. aureus, S. pyogenes et E. faecium.

## Revendications

1. Sous toutes les formes isomères possibles ainsi que leurs mélanges, les composés de formule (I) : 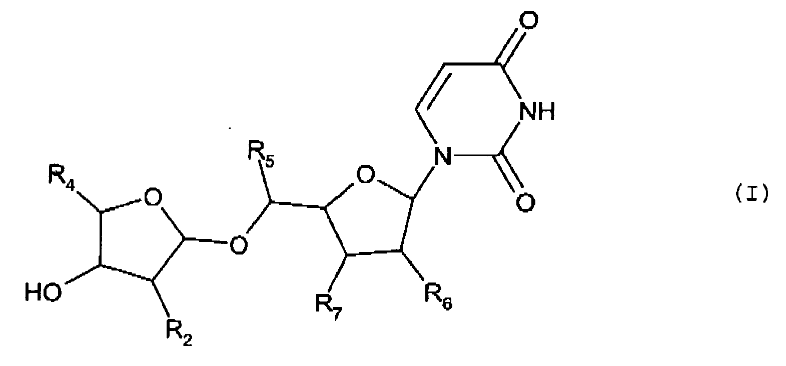
dans lesquels R₂ représente un atome d'hydrogène ou d'halogène, un radical alkyle, alkényle ou alkynyle renfermant jusqu'à 12 atomes de carbone, un radical OH, O-alkyle, OCO-alkyle, renfermant jusqu'à 18 atomes de carbone, un radical O-aryle ou OCO-aryle renfermant jusqu'à 15 atomes de carbone,
R₄ représente un radical CH₂NH₂, CH₂NHalkyle renfermant jusqu'à 18 atomes de carbone, CH₂N(alkyle1)(alkyle2) dans lequel alkyle1 et alkyle2 identiques ou différents l'un de l'autre, représentent un radical alkyle linéaire ou ramifié, renfermant jusqu'à 18 atomes de carbone, ou R₄ représente un radical CH₂-guanidine ou CH₂-amidine, un radical CH₂- hétérocycle, ou CH₂-NH-hétérocycle ou CH₂-NH-aryle ou CH₂-NH-hétéroaryle éventuellement substitués,
R₅ représente un radical 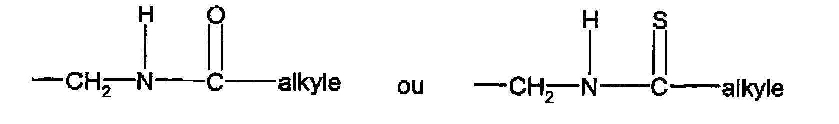 renfermant jusqu'à 24 atomes de carbone, un radical 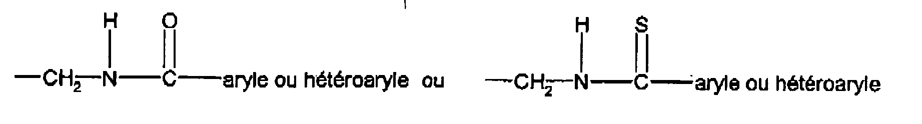 renfermant jusqu'à 24 atomes de carbone, un radical CH₂N₃, un radical : 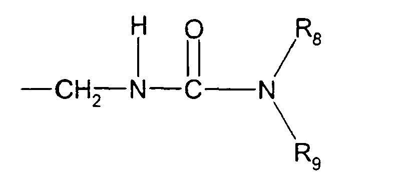 un radical : 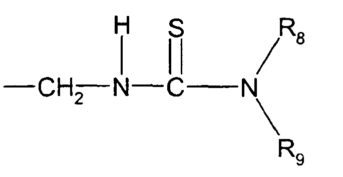 un radical : 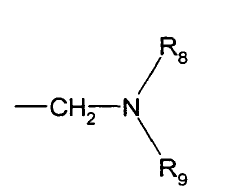
avec R₈ représentant un atome d'hydrogène ou un radical alkyle renfermant jusqu'à 4 atomes de carbone,
et R₉ représentant un radical alkyle, linéaire ou ramifié renfermant au plus 18 atomes de carbone, un radical carbocyclique ou hétérocyclique renfermant 5 ou 6 chaînons, un radical aryle ou hétéroaryle éventuellement substitués, R₈ et R₉ pouvant également constituer avec l'atome d'azote qui les porte un hétérocycle éventuellement substitué, ou R₅ représente un radical CH₂alkyle, CH₂-aryle ou CH₂-hétéroaryle éventuellement substitué, CH₂Oalkyle ou CO₂alkyle renfermant jusqu'à 18 atomes de carbone, ou CH₂S-alkyle renfermant jusqu'à 16 atomes de carbone, ou CH₂S-aryle ou CH₂S-hétéroaryle, éventuellement substitués,
R₆ représente un atome d'hydrogène ou d'halogène, un radical OH, Oalkyle ou OCOalkyle renfermant jusqu'à 18 atomes de carbone, un radical S-alkyle, S-aryle ou S-hétéroaryle renfermant jusqu'à 12 atomes de carbone,
tous les radicaux alkyle, aryle, hétéroaryle, les radicaux carbocycliques et hétérocycliques définis ci-dessus étant éventuellement substitués par un ou plusieurs radicaux choisis parmi les atomes d'halogène ; le radical hydroxyle ; les radicaux alkyle ou alcoxy linéaires ou ramifiés, renfermant 1 à 4 atomes de carbone ; le radical phényle et phénylalkyle, le radical phényle étant lui-même éventuellement substitué par un atome d'halogène ou un radical phényle et le radical alkyle linéaire ou ramifié renfermant de 1 à 4 atomes de carbone ; un radical carbocyclique renfermant 4 ou 6 chaînons ;
R₇ représente un atome d'hydrogène ou un radical OH libre, éthérifié ou estérifié, ainsi que les sels d'addition avec les acides des composés de formule (I).

2. Les composés définis à la revendication 1 de formule (IA) : 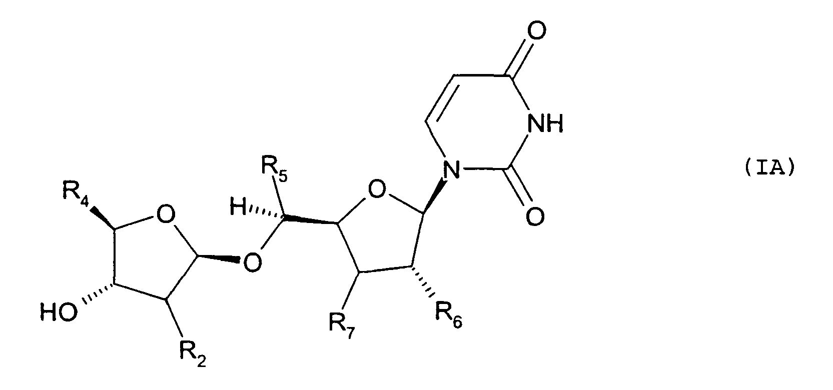 dans lesquels les divers substituants conservent la même signification que dans la revendication 1.

3. Les composés de formule (I) définis à la revendication 1 ou 2, dans lesquels R₇ représente un atome d'hydrogène.

4. Les composés de formule (I) définis à la revendication 1, 2 ou 3, dans lesquels R₆ représente un radical OH ou un atome de fluor.

5. Les composés de formule (I) définis à l'une quelconque des revendications 1 à 4 dans lesquels R₂ représente un radical OH.

6. Les composés de formule (I) définis l'une quelconque des revendications 1 à 4, dans lesquels R₂ représente un atome de fluor.

7. Les composés de formule (I) définis à l'une quelconque des revendications 1 à 6 dans lesquels R₄ représente un radical CH₂NH₂.

8. Les composés de formule (I) définis à l'une quelconque des revendications 1 à 6 dans lesquels R₄ représente un radical CH₂NHCH₃ ou CH₂NHC₂H₅.

9. Les composés de formule (I) définis à l'une quelconque des revendications 1 à 8 dans lesquels R₅ représente un radical

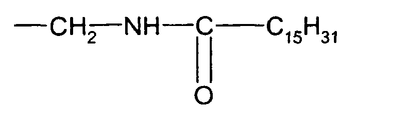

10. Les composés de formule (I) définis à l'une quelconque des revendications 1 à 8, dans lesquels R₅ représente un radical 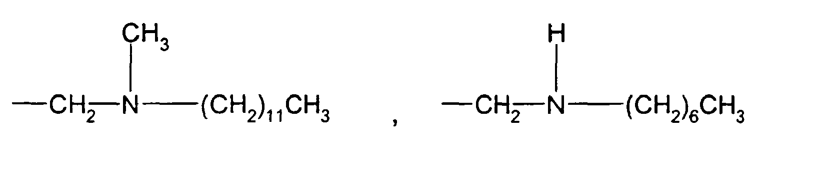 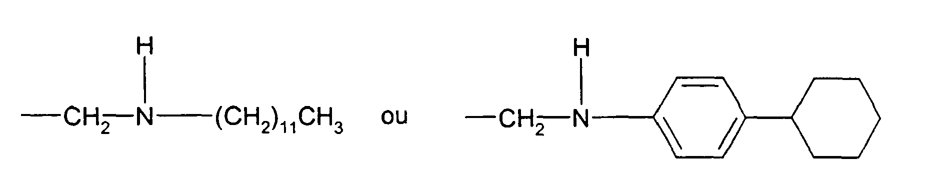 ou CH₂-NH-alkyle avec alkyle linéaire ou ramifié renfermant 8 à 14 atomes de carbone.

11. Les composés de formule (I) définis à la revendication 1 dont le nom suit :
- 1-[5-O-(5-amino-5-déoxy-.bêta.-D-ribofuranosyl)-3,6-didéoxy-6-[(1-oxohexadécyl)amino]-.bêta.-D-ribohexofuranosyl]-2,4-(1H,3H)-pyrimidinedione.

12. Les compositions pharmaceutiques renfermant comme médicaments au moins un composé de formule (I) défini à l'une des revendications 1 à 11, à titre de principe actif, ou l'un de ses sels pharmaceutiquement acceptables.

13. Procédé de préparation des composés de formule (I) défini à l'une quelconque des revendications 1 à 11, **caractérisé en ce que** l'on soumet un composé de formule (II) : 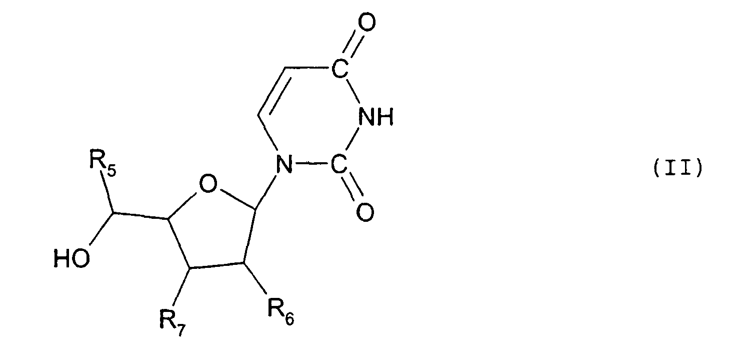 dans laquelle les divers substituants conservent leur signification précédente, à l'action d'un composé de formule (III) : 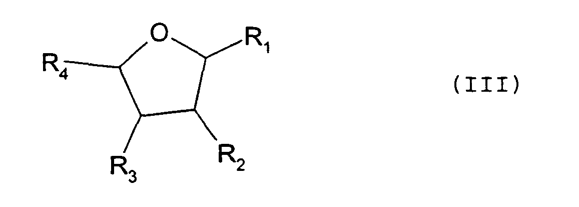 dans laquelle R₁ représente un atome d'halogène ou un radical OC(NH)C(Hal)₃ avec Hal représentant un atome d'halogène ou un radical 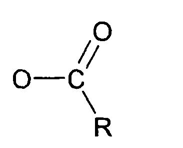 avec R représentant un radical alkyle renfermant jusqu'à 18 atomes de carbone ou OC(NH)C(Hal)₃, Hal représentant un atome d'halogène, les autres substituants R₂, R₃ et R₄ conservent leur signification précédente pour obtenir le composé de formule (I) correspondant que l'on soumet si désiré à une ou plusieurs des opérations suivantes : addition, substitution, condensation et salification pour obtenir le composé de formule (I) recherché.

14. A titre de produits chimiques nouveaux les composés de formule (II) définis à la revendication 13.

## Claims

1. In all possible isomer forms as well as their mixtures, the compounds of formula (I): 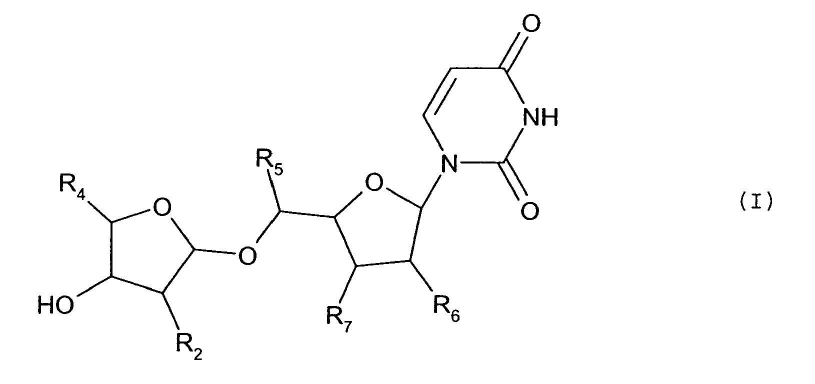
in which R₂ represents a hydrogen or halogen atom, an alkyl, alkenyl or alkynyl radical containing up to 12 carbon atoms, an OH, O-alkyl, OCO-alkyl radical containing up to 18 carbon atoms, an O-aryl or OCO-aryl radical containing up to 15 carbon atoms,
R₄ represents a CH₂NH₂, CH₂NHalkyl radical containing up to 18 carbon atoms, a CH₂N(alkyl1)(alkyl2) radical in which alkyl1 and alkyl2 identical to or different from one another represent a linear or branched alkyl radical containing up to 18 carbon atoms, or R₄ represents a CH₂-guanidine radical or a CH₂-amidine, a CH₂- heterocycle, CH₂-NH-heterocycle or CH₂-NH-aryl or CH₂-NH-heteroaryl radical optionally substituted,
R₅ represents a 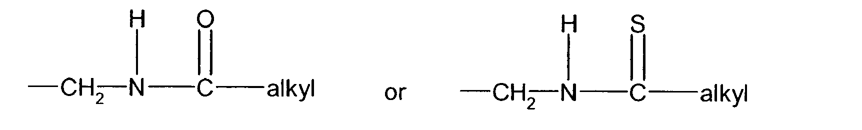 radical containing up to 24 carbon atoms, a 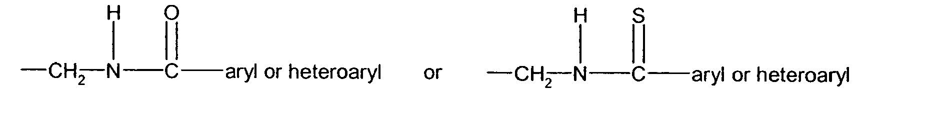 radical containing up to 24 carbon atoms, a CH₂N₃ radical, 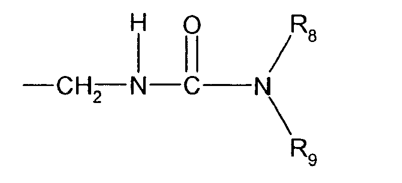 radical
a: 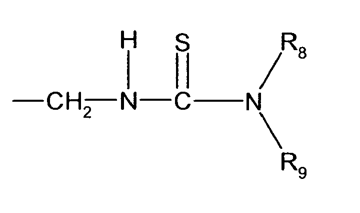 radical
a: 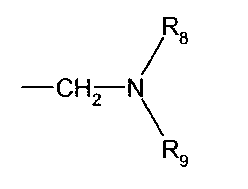 radical
with R₈ representing a hydrogen atom or an alkyl radical containing up to 4 carbon atoms,
and R₉ representing an alkyl radical, linear or branched containing at most 18 carbon atoms, a carbocyclic or heterocyclic radical containing 5 or 6 members, an aryl or heteroaryl radical optionally substituted, R₈ and R₉ also being able to form with the nitrogen atom which carries them an optionally substituted heterocycle, or R₅ represents a CH₂alkyl, CH₂-aryl or CH₂-heteroaryl radical optionally substituted, CH₂Oalkyl or CO₂alkyl radical containing up to 18 carbon atoms, or CH₂S-alkyl radical containing up to 16 carbon atoms, or CH₂S-aryl or CH₂S-heteroaryl radical optionally substituted,
R₆ represents a hydrogen or halogen atom, an OH, O-alkyl or OCO-alkyl radical containing up to 18 carbon atoms, an S-alkyl, S-aryl or S-heteroaryl radical containing up to 12 carbon atoms,
all the alkyl, aryl, heteroaryl radicals, the carbocyclic and heterocyclic radicals defined above being optionally substituted by one or more radicals chosen from the halogen atoms; the hydroxyl radical; the linear or branched alkyl or alkoxy radicals containing 1 to 4 carbon atoms; the phenyl and phenylalkyl radical, the phenyl radical itself being optionally substituted by a halogen atom or a phenyl radical and the linear or branched alkyl radical containing 1 to 4 carbon atoms; a carbocyclic radical containing 4 or 6 members;
R₇ represents a hydrogen atom or a free, etherified or esterified OH radical,
as well as the addition salts with acids of the compounds of formula (I) .

2. The compounds defined in claim 1 of formula (IA): 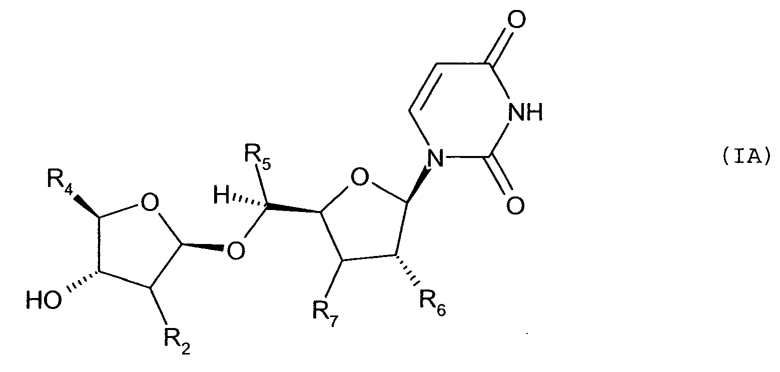 in which the various substituents retain the same meaning as in claim 1.

3. The compounds of formula (I) defined in claim 1 or 2, in which R₇ represents a hydrogen atom.

4. The compounds of formula (I) defined in claim 1, 2 or 3, in which R₆ represents an OH radical or a fluorine atom.

5. The compounds of formula (I) defined in any one of claims 1 to 4 in which R₂ represents an OH radical.

6. The compounds of formula (I) defined in any one of claims 1 to 4, in which R₂ represents a fluorine atom.

7. The compounds of formula (I) defined in any one of claims 1 to 6 in which R₄ represents a CH₂NH₂ radical.

8. The compounds of formula (I) defined in any one of claims 1 to 6 in which R₄ represents a CH₂NHCH₃ or CH₂NHC₂H₅ radical.

9. The compounds of formula (I) defined in any one of claims 1 to 8 in which R₅ represents a 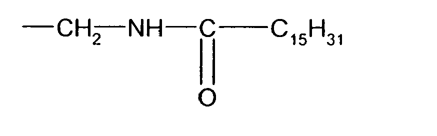 radical.

10. The compounds of formula (I) defined in any one of claims 1 to 8, in which R₅ represents a 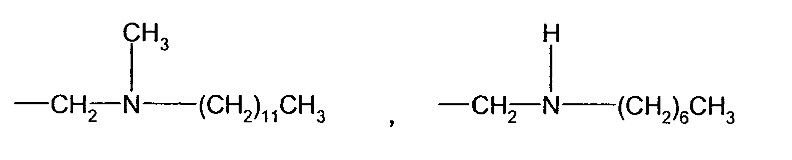 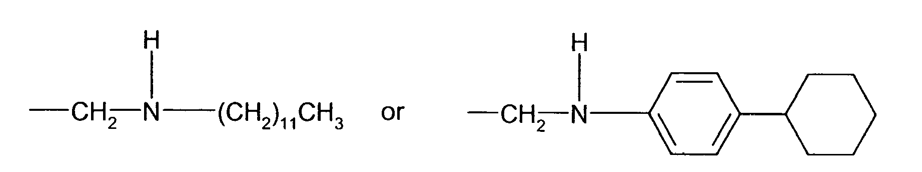 or CH₂-NH-alkyl radical with linear or branched alkyl containing 8 to 14 carbon atoms.

11. The compounds of formula (I) defined in claim 1 the name of which follows:
- 1-[5-O-(5-amino-5-deoxy-.beta.-D-ribofuranosyl)-3,6-dideoxy-6-[(1-oxohexadecyl)amino]-.beta.-D-ribohexofuranosyl]-2,4-(1H,3H)-pyrimidinedione.

12. The pharmaceutical compositions containing as medicaments at least one compound of formula (I) defined in one of claims 1 to 11, as active ingredient, or one of its pharmaceutically acceptable salts.

13. Process for the preparation of the compounds of formula (I) defined in any one of claims 1 to 11, **characterized in that** a compound of formula (II): 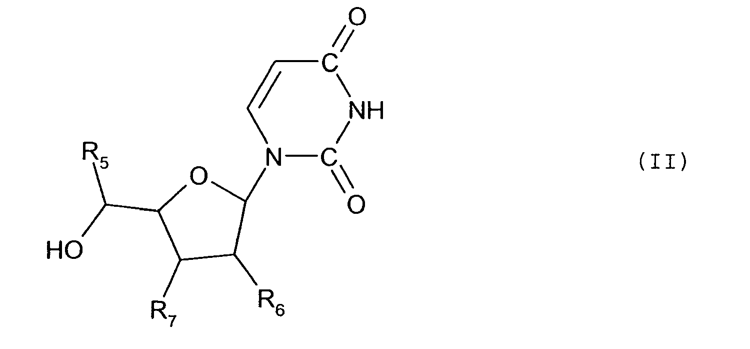 in which the various substituents retain their previous meaning, is subjected to the action of a compound of formula (III) : 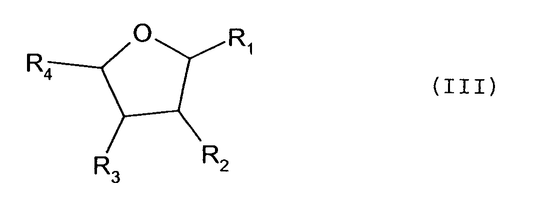 in which R₁ represents a halogen atom or an OC(NH)C(Hal)₃ radical with Hal representing a halogen atom or a 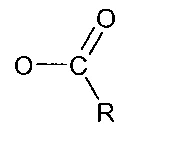 radical with R representing an alkyl radical containing up to 18 carbon atoms or OC(NH)C(Hal)₃, Hal representing a halogen atom, the other substituents R₂, R₃ and R₄ retain their previous meaning in order to obtain the corresponding compound of formula (I) which is subjected, if desired, to one or more of the following operations: addition, substitution, condensation and salification in order to obtain the sought compound of formula (I).

14. As new chemical products the compounds of formula (II) defined in claim 13.

## Patentansprüche

1. Verbindungen der Formel (I) in all ihren möglichen isomeren Formen sowie ihren Gemischen: 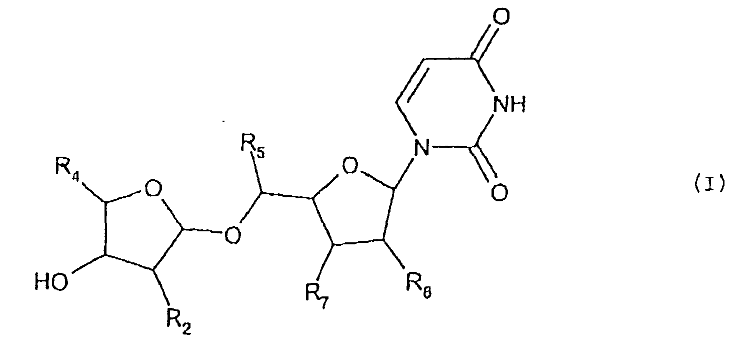
worin R₂ für ein Wasserstoffatom oder Halogenatom, einen Alkyl-, Alkenyl-, oder Alkinylrest, der bis zu 12 Kohlenstoffatome umfasst, einen OH-, O-Alkyl-, OCO-Alkyl-Rest, der bis zu 18 Kohlenstoffatome umfasst, einen O-Aryl- oder OCO-Aryl-Rest, der bis zu 15 Kohlenstoffatome umfasst, steht;
R₄ für einen Rest CH₂NH₂, CH₂NH-Alkyl, der bis zu 18 Kohlenstoffatome umfasst, CH₂N(Alkyl1)(Alkyl2), worin Alkyl1 und Alkyl2, die gleich oder verschieden voneinander sind, einen linearen oder verzweigten Alkylrest darstellen, der bis zu 18 Kohlenstoffatome umfasst, steht, oder R₄ einen CH₂-Guanidin- oder CH₂-Amidin-Rest, einen CH₂-Heterocyclyl-Rest oder CH₂-NH₂-Heterocyclyl oder CH₂-NH₂-Aryl- oder CH₂₋NH-Heteroaryl-Rest, die gegebenenfalls substituiert sind, darstellt;
R₅ einen Rest 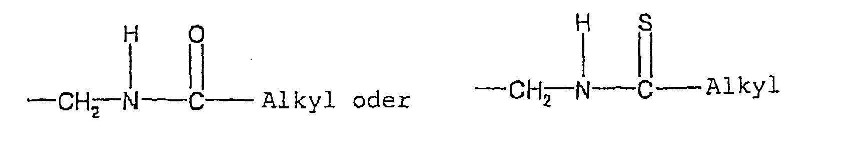 der bis zu 24 Kohlenstoffatome umfasst, einen Rest 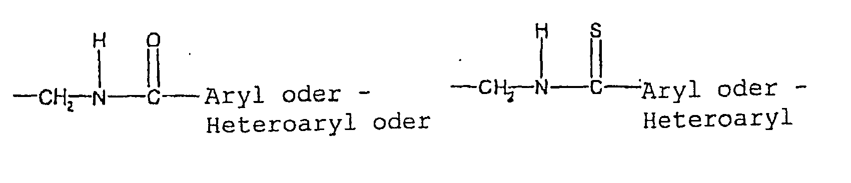 der bis zu 24 Kohlenstoffatome umfasst, einen Rest CH₂N₃, einen Rest: 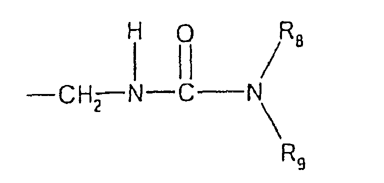 einen Rest: 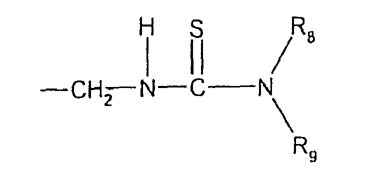 einen Rest: 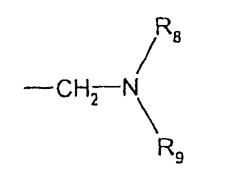 darstellt, wobei R₈ ein Wasserstoffatom oder einen Alkylrest, der bis zu 4 Kohlenstoffatome umfasst, darstellt und R₉ einen linearen oder verzweigten Alkylrest, der höchstens 18 Kohlenstoffatome umfasst, einen carbocyclischen oder heterocyclischen Rest, der 5 oder 6 Ringglieder umfasst, einen gegebenenfalls substituierten Aryl- oder Heteroarylrest darstellt, wobei R₈ und R₉ auch mit dem Stickstoffatom, das sie trägt, einen gegebenenfalls substituierten Heterocyclus bilden können oder R₅ einen CH₂-Alkyl-, CH₂-Aryl- oder CH₂₋Heteroarylrest, der gegebenenfalls substituiert ist, CH₂O-Alkyl- oder CO₂-Alkyl-Rest, der bis zu 18 Kohlenstoffatome · umfasst, oder einem CH₂S-Alkyl-Rest, der bis zu 16 Kohlenstoffatome umfasst, oder CH₂S-Aryl- oder CH₂S-HeteroarylRest, der gegebenenfalls substituiert ist, darstellt;
R₆ ein Wasserstoffatom oder Halogenatom darstellt, einen OH-, O-Alkyl- oder OCO-Alkyl-Rest, der bis zu 18 Kohlenstoffatome darstellt, einen S-Alkyl-, S-Aryl- oder S-Heteroarylrest, der bis zu 12 Kohlenstoffatome umfasst, darstellt, wobei alle Alkyl-, Aryl-, Heteroaryl-Reste, carbocyclischen und heterocyclischen Reste, die oben definiert sind, gegebenenfalls mit einem oder mehreren Resten substituiert sind, ausgewählt unter Halogenatomen; Hydroxylrest, linearen oder verzweigten Alkyl- oder Alkoxyresten, die 1 bis 4 Kohlenstoffatome umfassen; dem Phenyl- und Phenylalkylrest, wobei der Phenylrest selbst gegebenenfalls mit einem Halogenatom oder einem Phenylrest substituiert ist und der lineare oder verzweigte Alkylrest 1 bis 4 Kohlenstoffatome umfasst; einem carbocyclischen Rest, der 4 oder 6 Kettenglieder umfasst;
R₇ ein Wasserstoffatom oder einen OH-Rest, der frei, verethert oder verestert ist, darstellt,
sowie die Säureadditionssalze der Verbindungen der Formel (I).

2. Verbindungen, wie sie nach Anspruch 1 definiert sind, mit der Formel (IA): 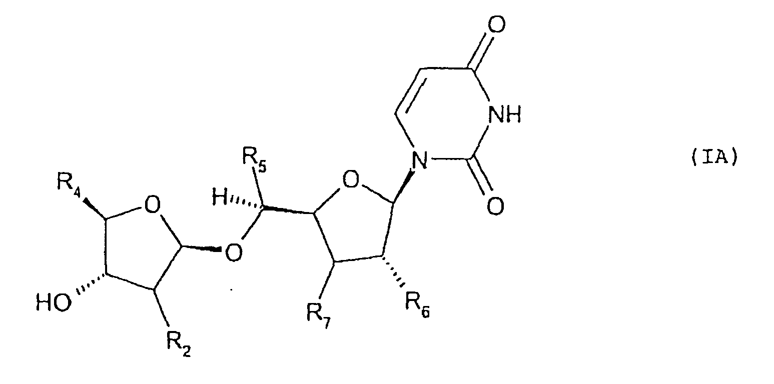 worin die verschiedenen Substituenten dieselbe Bedeutung wie in Anspruch 1 beibehalten.

3. Verbindungen der Formel (I), wie sie in Anspruch 1 oder 2 definiert sind, wobei R₇ ein Wasserstoffatom darstellt.

4. Verbindungen der Formel (I), wie sie in Anspruch 1, 2 oder 3 definiert sind, wobei R₆ einen OH-Rest oder ein Fluoratom darstellt.

5. Verbindungen der Formel (I), wie sie in einem der Ansprüche 1 bis 4 definiert sind, wobei R₂ einen OH-Rest darstellt.

6. Verbindungen der Formel (I), wie sie in einem der Ansprüche 1 bis 4 definiert sind, wobei R₂ ein Fluoratom darstellt.

7. Verbindungen der Formel (I), wie sie in einem der Ansprüche 1 bis 6 definiert sind, wobei R₄ einen Rest CH₂NH₂ darstellt.

8. Verbindungen der Formel (I), wie sie in einem der Ansprüche 1 bis 6 definiert sind, wobei R₄ einen Rest CH₂NHCH₃ oder CH₂NHC₂H₅ darstellt.

9. Verbindungen der Formel (I), wie sie in einem der Ansprüche 1 bis 8 definiert sind, wobei R₅ einen Rest 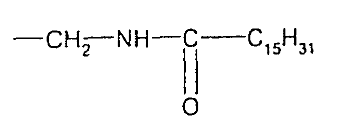 darstellt.

10. Verbindungen der Formel (I), wie sie in einem der Ansprüche 1 bis 8 definiert sind, wobei R₅ einen Rest 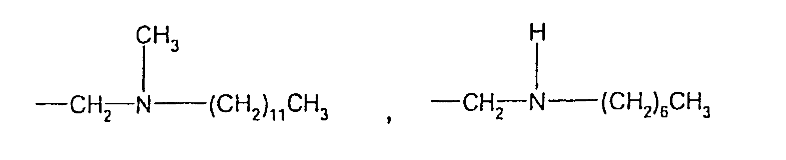 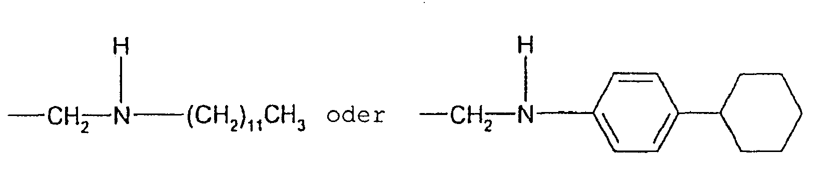 oder CH₂-NH-Alkyl, wobei Alkyl linear oder verzweigt ist und 8 bis 14 Kohlenstoffatome umfasst, darstellt.

11. Verbindungen der Formel (I), wie sie in Anspruch 1 definiert sind, mit dem Namen: 1-[5-O-(5-Amino-5-desoxy-beta-D-ribofuranosyl)-3,6-didesoxy-6-[(1-oxohexadecyl)amino]-beta-D-ribohexofuranosyl]-2,4-(1H,3H)-pyrimidindion.

12. Pharmazeutische Zusammensetzungen, die als Medikamente wenigstens eine Verbindung der Formel (I), wie sie in einem der Ansprüche 1 bis 11 definiert ist, als Wirkstoff oder eines ihrer pharmazeutisch annehmbaren Salze umfassen.

13. Verfahren zu Herstellung der Verbindungen der Formel (I), wie sie in einem der Ansprüche 1 bis 11 definiert sind, **dadurch gekennzeichnet, dass** man eine Verbindung der Formel (II): 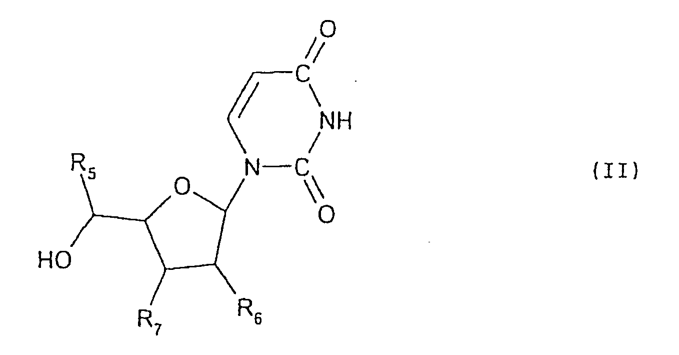 in der die verschiedenen Substituenten ihre vorstehend genannte Bedeutung beibehalten, der Wirkung einer Verbindung der Formel (III): 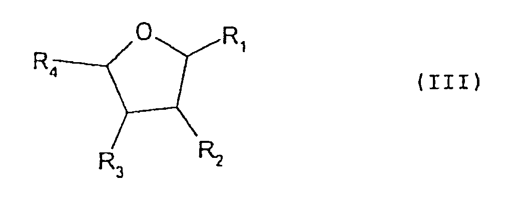 unterwirft, worin R₁ ein Halogenatom oder einen Rest OC(NH)C(Hal)₃, worin Hal ein Halogenatom darstellt, oder einen Rest, 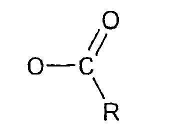 worin R einen Alkylrest, der bis zu 18 Kohlenstoffatome umfasst, oder C(NH)C(Hal)₃ darstellt, worin Hal für ein Halogenatom steht, die anderen Substituenten R₂, R₃ und R₄ ihre vorstehende Bedeutung beibehalten, unter Erhalt der entsprechenden Verbindung der Formel (I), die man, wenn es gewünscht wird, einem oder mehreren der folgenden Verfahrensschritte unterwirft:
Addition, Substitution, Kondensation und Salzbildung, um die gesuchte Verbindung der Formel (I) zu erhalten.

14. Verbindungen der Formel (II), wie sie in Anspruch 13 definiert sind, als neue chemische Produkte.
